Europäisches Patentamt

(19) European Patent Office

Office européen des brevets

(11) Publication number: **0 246 192**
**A2**

# (12) EUROPEAN PATENT APPLICATION

(21) Application number: **87810291.2**

(22) Date of filing: **08.05.87**

(51) Int. Cl.⁴: **G 01 N 33/72**

(30) Priority: **16.05.86 US 864182**

(43) Date of publication of application:
**19.11.87 Bulletin 87/47**

(84) Designated Contracting States: **DE FR GB NL SE**

(71) Applicant: **WARNER-LAMBERT COMPANY**
**201 Tabor Road**
**Morris Plains New Jersey 07950 (US)**

(72) Inventor: **Talmage, Joseph M.**
**693 Reba Road**
**Landing§New Jersey 07850 (US)**

(74) Representative: **Silbiger, Jakob, Dr.**
**c/o CAPSUGEL AG Münchensteinerstrasse 41**
**CH-4002 Basel (CH)**

(54) Occult blood test monitor.

(57) An in-bowl test and method for making which includes a particulate peroxide generating agent in PVP in combination with an oxygen sensitive dye such as guaiac.

EP 0 246 192 A2

Bundesdruckerei Berlin

**Description**

OCCULT BLOOD TEST MONITOR

This invention relates to a testing monitor useful for detection of peroxidase-like activity and, in particular, a test kit containing a testing monitor for detection of such activity in stool samples.

The detection of peroxidase-like activity has found to be indicative of the presence of hemoglobin in a specimen and, reported to be correlated to the heightened fertility period in females during the estrous cycle. The detection of peroxidase-like activity has, therefore, become an invaluable aid to the medical practitioner.

Over 100,000 persons in the United States are affected by cancer of the colon and rectum each year. When the number of colorectal cancers occurring each year is combined with the number of cancers occurring in other digestive organs, including the esophagus and stomach, such cancers of the digestive system account for more occurrences of cancer than any other single form of the disease.

Contrary to many other forms of cancer, early diagnosis and treatment of digestive tract cancer results in a cure rate of 80 to 90% of those affected. This cure rate drastically decreases as cancer reaches its later stages. Thus, early detection of the disease is critical to successful treatment.

Most, but not all cancers of the digestive tract bleed to a certain extent. Blood found in the gastric contents and in vomitus is indicative to conditions associated with disorders of the mucous membrane, such as ulcers, diverticulitis, colitis and carcinoma. In addition, blood is also deposited in fecal matter extruded from the digestive system. The presence of blood in fecal matter is not normally detected, however, until gross bleeding occurs. When this is evident, digestive tract cancers are in their advanced stages.

The general reactants involved in the occult blood detection test are well known and include an oxygen sensitive dye and a peroxygen source which liberates hydrogen peroxide and in the presence of haem oxidizes the dye. The detection of peroxidase activity has become an invaluable aid to the medical practitioner for the diagnosis of a number of disorders.

Any of the oxygen sensitive dye components customarily used in these types of indicator reactions are suitable and they include for example, gum guaiac and its derivatives, aniline and its derivatives, o-tolidine, o-toluidine, p-toluidine, benzidine, tetramethylbenzidene, di-anisidine, o-cresol, m-cresol, alpha naphthol, beta naphthol, catechol, guaiacol, pyrogallol and mixtures thereof.

The preferred indicator material is guaiac because of its recognition as an effective indicator, its non-carciogenicity and commercial availability. In the presence of an oxygen source and peroxidase-like activity, gum guaiac changes from colorless to a blue color. One of the most widely used oxygen sensitive reagents for diagnosing occult-blood is derived from an extract from the wood of certain species of trees of the Guaiacum genus native to the American tropics. The extract, termed guaiac, turns from essentially colorless to blue in the presence of hemoglobin and a oxidizing source such as hydrogen peroxide. More specifically, the guaiac reagent is sensitive to what is termed "peroxidase activity" which results from the combination of an oxidizing agent with hemoglobin or certain chemically similar compounds.

Various test equipment and procedures have been developed for use by physicians in testing for the presence of occult blood in fecal matter. One of the most successful tests is manufactured and sold by Smith Kline Diagnostics of Sunnyvale, California under the trademark "Hemoccult." The package for the Hemoccult test is disclosed in U.S. Patent 3,996,006 issued to J. Pagano. Briefly, the Pagano test employs an absorbent white paper impregnated with a guaiac reagent and encased in a special test slide having openable flaps on both sides. To use the Pagano test slide, one must obtain a sample of fecal matter, smear it onto the guaiac-impregnated paper by opening the panel on one side of the test slide, and then close the panel. A panel on the opposite side of the test slide is then opened and contacted with a developing agent. The developing agent is a stabilized solution of hydrogen peroxide and denatured alcohol which is applied to the guaiac-impregnated paper. If occult blood is present in the fecal matter smeared on the opposite side of the paper, the product of the guaiac reaction will appear as a blue substance against the white paper background, providing a positive indication of the presence of blood in the fecal matter. The Pagano test is designed for evaluation by a diagnostic laboratory. As a test which can be evaluated at home, the Pagano test has two shortcomings. First, it requires the person to handle fecal matter and secondly, it requires the tester to be somewhat sophisticated when interpretating the results.

There is another problem inherent in the Pagano test and other tests which require laboratory interpretation. This problem relates to the difference in time between the taking of the sample and its evaluation. Of course, there is the obvious problem of storing the test kit after the sample has been taken and, since it is desirable to minimize the premature decomposition of the reactants, the sample must be kept cold. Refrigerator storage of fecal samples is obviously unpopular when the refrigerator storage space must be shared by food utilized by the consumer.

A more serious problem with clinical tests is disclosed in "Gastroenterology 1982": 1982-986-91 in which it is stated that prolonged storage in desiccation can affect the reliability of the guaiac-impregnated slide test in detecting fecal occult blood. The reliability of the test is related to hemoglobin concentration; a slightly positive reaction can become negative upon storage and a test yielding strong positivity for blood can convert to less pronounced or even ambiguous results. The exact reason for this apparent time dependent conversion is not known, although it is assumed that the denaturation of hemoglobin in stool by proteolytic enzymes and bacteria occurs. It has been shown by this reference that a concentration of hemoglobin and stool may produce a very strong positive reaction which is stable for thirty days whereas a weak positive response will become negative on the sixth day after specimen application. For these reasons, it is apparent that a test which can be conducted reliably by the individual in his own home and which can yield readily discernably results is the test method of choice.

A non-clinical test is disclosed by D. E. Fonner in U.S. Patent No. 2,838,377. The Fonner test utilizes O-tolidine and benzidine. The reagents when mixed with blood and other reactants produce a visible dye. The Fonner test employs a testing apparatus which can be dropped into the toilet bowl. The Fonner test has, however, not been particularly successfully because of the high rate of false positive indications associated with it. U.S. Patent 4,541,987 issued to P.A. Guadagno also discloses an in-bowl test utilizing guaiac instead of o-tolidine.

In-bowl tests have an inherent advantage over test such as described in the Pagano patent, in that they do not require fecal sample collection, the reactants being added to the sample in the bowl.

In-bowl testing however also has some disadvantages associated with it. First, the reaction itself must be visible in the toilet bowl containing fecal matter. Secondly, since the haem necessary for generating the reaction is present initially in the stool, the in-bowl reaction must be sensitive enough to react with the haem, that is leached out in the toilet water. Also, since the reaction must have this increasing sensitivity, it is necessary that each of the reactive components be present in precisely predetermined levels and each of these reactants must be fully reactive, and not be prematurely reacted.

Since, during the reaction itself the peroxygen source and the oxygen sensitive dye must be in reactive contact, the inhibition of the premature reaction poses a problem.

Guadagno teaches using a particulate peroxygen compound such as "Oxone" sold by DuPont Co. and comprises 2 moles of potassium monopersulfate, one mole of potassium hydrogen sulfate and one mole of potassium sulfate. Particulate inorganic salts capable of generating hydrogen peroxide when reacted with water are advantageous for in the bowl tests because they are substantially inert when dry.

To react successfully with the chromogen however, there must be ample particulate peroxygen in reactive contact to produce the desired observable color change when the test is performed.

The question then becomes how is reactive contact maintained between the components when needed for the test but without producing a premature reaction.

Since particulate inorganic peroxygen, liberates hydrogen peroxide when dissolved in water, they cannot be coated on a guaiac-containing substrate from an aqueous solution. These particulates may be physically entrapped between sheets of filter paper. This system is disclosed in Wells European Patent Application No. 82307021.4. Alternatively it may be dusted on a sheet of bibulous paper upon which a coating of guaiac which is water insoluble as dissolved in a suitable organic solvent and a film former such as polyethylene glycol (PEG) or polyvinylpyrollidone (PVP) has been cosolubilized such as described in Wells European Patent Application No. 82307021.4. Svoboda U.S. Patent 3,975,161 also uses PVP and PEG or other film-formers to protect chromogens other than guaiac from premature reaction in an occult blood test.

Each of these methods of entrapping reactants have disadvantages, however.

Entrapment between sheets of filter paper means that the reaction occurs inside the filter paper which requires that the haem in solution penetrate the paper. This produces a time delay and detracts from the sensitivity of the reaction because the blue color must migrate outward through the filter paper, then dye the external paper surface with sufficient intensity to be observed by an inexperienced user.

Dusting on a previously coated substrate has disadvantages also. Since the substrate is absorbent, there is little residual tack to capture the particulate peroxygen. Also, the surface is likely to be discontinuous due to differences in absorption of the film-former solution. Furthermore, agitation of the coated substrate during transit, containing the dusted particulate, is likely to dislodge the particles. At the least, these problems affect the uniformity of the reaction across the surface of a bibulous carrier, and obviously also affects the reaction sensitivity when less than a stoichrometric amount of peroxygen is present.

According to this invention an in the bowl occult blood test having superior predictability and sensitivity is provided by dissolving or suspending a particulate peroxygen compound in a mixture of a suitable organic suspension aid and PVP, and coating this suspension on a bibulous substrate previously impregnated with guaiac.

Also contemplated is an in-bowl test having particulate peroxygen essentially uniformly dispersed throughout a matrix comprising PVP and in suitable proximity with guaiac for a color changing reaction to occur in the presence of occult blood.

This invention enables the production of an in-bowl occult blood test with precise control of the sensitivity of the indicator reaction.

While deposition of the particulate peroxide generating agents on a guaiac impregnated substrate by means of any film former can produce an essentially uniform distribution of particles and insure that sufficient particles are present for the indicator reaction to occur, it has been discovered that only PVP is resistant to reaction with the particles over extended periods of storage, thereby allowing a uniform dispersion of reactant peroxygen or the substrate as well as a storage stable product.

Since, in the method of this invention, PVP with the particles suspended within is applied to a surface containing guaiac, the PVP will also protect guaiac. Further, since the particulate is within the PVP film rather than dusted on the surface, uniform dispersion of particulates at predetermined levels is obtained at the time the test kit is made. If, on the other hand, the dusting of particulate occurs after the film former has set up the particulate is not embedded and can therefore be easily displaced.

The peroxygen source is selected from materials that will yield hydrogen peroxide in the presence of water.

Representative peroxygen sources include both organic and inorganic peroxides. Illustrative compounds include monopersulfates, cumene hydroperoxide, butyl hydroperoxide and mixtures thereof.

Currently a particularly preferred peroxide is sold by Dupont Company under the trademark "Oxone" which comprises two moles of potassium monopersulfate, one mole of potassium hydrogensulfate and one mole of potassium sulfate. The Oxone® suspension is prepared by suspending 25 to 250 milligrams per milliliter in a suitable organic solvent such as chloroform.

The particulate peroxygen source is dissolved or suspended in a PVP containing organic solvent for deposition on the matrix. A wide range of organic solvents may be used which are non-reactive with the formulation ingredients. Representative organic solvents include compounds selected from      1) ethers, such as diethyl ether, diisopropyl ether;

2) ketones, such as acetone, methylethylketone, methylisobutylketone;

3) aromatic hydrocarbons such as benzene and toluene;

4) chlorinated hydrocarbons such as methylene chloride, chloroform, and carbon tetrachloride,

PVP is mixed with "Oxone" or other particulates in a proportion of from 1:4 to 5:1, by weight with a ratio of 2:1 to 1:2 being preferred.

The organic solvent is added to create a uniform, free flowing suspension generally about 5 to 10 times the volume of PVP although the precise amount is not critical.

The preparation of guaiac solution formulations for deposition in the test matrix is governed by the absorbability of the particular matrix employed and upon the desired sensitivity, i.e., the extent of the color change desired for a particular level of peroxidase activity. Preferred useful amounts may range from 0.01% to about 3.0% and preferably about 0.25% to about 2.0% by weight based upon the weight of the entire formulation. Amounts above about 3% are not desired since they may result in hypersensitivity showing false positive results. Amounts below about 0.01% are not recommended since they do not provide sufficient color development for accurate detection of peroxidase-like activity.

A buffer is added to the guaiac formulation in solution prior to coating the paper to aid in stability and to provide the optimum pH value to enable catalytic activity to occur. It is essential that the buffer selected maintain the pH within a range in which the indicator material changes color upon oxidation. Normally this will be between a pH of about 2 and about 8 and preferably between about 3 and about 5. Higher pH values should be avoided to prevent autooxidation of the indicator, resulting in false positive results. Lower pH values inhibit efficient oxidation. Representative buffers include citrate, tartrate, phosphate, acetate and mixtures thereof with citrate buffers being preferred.

PVP may be added to this combination to act as an added protector for premature oxidation although the addition to a guaiac coated bibulous paper of the Oxone suspension containing PVP accomplishes the same thing.

It is also preferred to add a sequestrant such as EDTA to the guaiac to prevent metal catalysis of the reaction to produce a false positive. The sequestrant is added to a level of from 0.002 to about 0.6% by weight of the guaiac peroxygen combination.

The method for making the in-bowl test device of this invention is by impregnating a strip of a bibulous carrier-substrate with a guaiac solution, coating the impregnated carrier with a suspension of a particulate peroxygen source and PVP in a suitable carrier, thereby producing an essentially uniform dispersion coating of peroxygen on the surface of the carrier.

The carrier itself is flushable and preferably white to promote observation of the reaction and preferably has a tendency to float at least initially so that the reaction may be more easily observed. A suitable substrate is Whatman No. 1 filter paper.

The coated paper preferably has a delineated monitor test area on it for indicting the efficiacy of the reactants. This area has a haem compound added to it so that when it is wet a blue color will appear if the reagents are efficacious. Hemoglobin or other peroxidase activator is the catalytic agent that promotes the reaction between the indicator and the peroxygen source to produce the indicator color change. Such a monitor is described in Friend U.S. Patent 4,175,923.

Inventive and Comparative Example

Several film formers were dissolved in chloroform as shown in the Table below. (The differing percentages of solute represent a visual adjusting of solution viscosity to correspond to the viscosity of the preferred 5% PVP solution.)

## TABLE

| Solute Film Former | % by wt. |
|---|---|
| Polyethylene glycol (8000 M.) | 5.0 |
| Ethyl cellulose | 1.25 |
| Polyethylenepolypropylene glycol | 5.0 |
| Hydroxypropyl cellulose | 2.0 |
| Polyoxyethylene stearyl ether | 5.0 |
| PVP | 5.0 |

One gram of Oxone was added to 10ml of each of these solutions and agitated to form a suspension. These suspensions were then each swabbed on a piece of Whatman No. 1 filter paper impregnated with guaiac. When these coated treated papers were contacted with a standard haem solution, each produced a pronounced color change except for the hydroxypropyl cellulose coating which was essentially water insoluble and showed no reaction.

Duplicate samples of all of the film formers except hydroxypropyl cellulose were prepared, applied to paper previously coated with guaiac and stored at 60°C for four days. When contacted with haem solutions only PVP showed a color reaction identical to the initial test while the others all had substantially diminished intensity of indicator color. This surprising lack of stability clearly renders the other conventional film formers impractical for long term shelf stable storage required for this consumer performed test.

The in the bowl test of this invention provides superior sensitivity and predictability when compared to other in the bowl tests. Other variations within the ambit of this invention will readily occur to those skilled in the art and are within the scope of this invention.

**Claims**

I. A method of producing a test material for making an in bowl test for occult blood comprising coating a suspension of a particulate peroxide generating agent in PVP to a bibulous carrier containing a previously deposited oxygen sensitive indicator.

2. The method of claim I wherein the indicator is guaiac.

3. The method according to the claims I or 2 wherein the particulate is an inorganic salt capable of generating peroxide when contacted with water.

4. The method according to anyone of the claims I or 3, wherein the peroxide generator is at least one member selected from the group consisting of potassium sulfate, potassium monopersulfate, potassium hydrogensulfate.

5. A method of producing a test material for making an in bowl test for occult blood which comprises:
   a) contacting a bibulous carrier with a guaiac containing solution;
   b) drying the solution to fix guaiac on said carrier;
   c) suspending an inorganic particulate salt capable of generating peroxide in PVP solution;
   d) depositing said suspension on said carrier; and
   e) drying said suspension.

6. An in bowl test kit for occult blood comprising peroxide generating particulates in PVP in combination with an oxygen sensitive indicator.

7. The test kit of claim 6 wherein the indicator is guaiac.

8. The test kit according to the claims 6 or 7 wherein the particulate is an inorganic salt.

9. The test kit according to the claims 6 to 8 wherein the particulate is "Oxone".

10. The test kit of claims 6, 7, 8 or 9 wherein a bibulous carrier has the oxygen sensitive indicator deposited thereupon.

II. An in bowl test material for occult blood comprising a bibulous carrier having guaiac deposited thereupon and a coating of a particulate inorganic salt capable of generating peroxide dispersed in PVP overlaying said indicator deposited carrier.